# EUROPÄISCHE PATENTSCHRIFT

(11) **EP 3 296 315 B1**
(45) Veröffentlichungstag und Bekanntmachung des Hinweises auf die Patenterteilung: **08.05.2019**
(21) Anmeldenummer: 17001677.8
(22) Anmeldetag: 10.09.2013
(51) Int. Cl.: C07K 7/06, G01N 33/68

(54) **PEPTIDE ZUR BEHANDLUNG UND FRÜHDIAGNOSE VON MORBUS ALZHEIMER UND ANDERER TAUOPATHIEN**
PEPTIDES FOR THE TREATMENT AND EARLY DIAGNOSIS OF ALZHEIMER'S DISEASE AND OTHER TAUOPATHIES
PEPTIDE POUR LE TRAITEMENT ET LE DIAGNOSTIC PRÉCOCE DE LA MALADIE D'ALZHEIMER ET D'AUTRES TAUOPATHIES

(30) Priorität: 05.10.2012 DE 102012019882
(43) Veröffentlichungstag der Anmeldung: 21.03.2018
(62) Teilanmeldung aus: 13789476.2
(73) Patentinhaber: Forschungszentrum Jülich GmbH, 52425 Jülich (DE)
(72) Erfinder: Willbold, Dieter, 52428 Jülich (DE); Funke, Susanne Aileen, 96242 Sonnefeld (DE)

(56) Entgegenhaltungen:
- WO-A2-01/18546
- US-A1- 2010 204 085
- SIEVERS STUART A ET AL: "Structure-based design of non-natural amino-acid inhibitors of amyloid fibril formation", NATURE: INTERNATIONAL WEEKLY JOURNAL OF SCIENCE (AND SUPPLEMENTARY INFORMATION), NATURE PUBLISHING GROUP, UNITED KINGDOM, Bd. 475, Nr. 7354, 1. Juli 2011 (2011-07-01), Seiten 96-100, XP001526382, ISSN: 0028-0836, DOI: 10.1038/NATURE10154
- JING ZHENG ET AL: "Macrocyclic [beta]-Sheet Peptides That Inhibit the Aggregation of a Tau-Protein-Derived Hexapeptide", JOURNAL OF THE AMERICAN CHEMICAL SOCIETY, Bd. 133, Nr. 9, 9. März 2011 (2011-03-09), Seiten 3144-3157, XP055095036, ISSN: 0002-7863, DOI: 10.1021/ja110545h
- CHISATO NISHIURA ET AL: "Importance of Tyr310 residue in the third repeat of microtubule binding domain for filament formation of tau protein", JOURNAL OF BIOCHEMISTRY, Bd. 147, Nr. 3, 1. März 2010 (2010-03-01), Seiten 405-414, XP55426653, GB ISSN: 0021-924X, DOI: 10.1093/jb/mvp181

### SEQUENCE LISTING

<110> Forschungszentrum Jülich GmbH
<120> Peptide zur Behandlung und Frühdiagnose von Morbus Alzheimer und
   anderen Tauopathien
<130> PT 1.2590 PCT
<160> 4
<170> PatentIn version 3.5
<210> 1
   <211> 12
   <212> PRT
   <213> Artificial Sequence
<220>
   <223> D-Peptid (TD28)
<400> 1
   Thr Thr Ser Leu Gln Met Arg Leu Tyr Tyr Pro Pro
   1               5                   10
<210> 2
   <211> 12
   <212> PRT
   <213> Artificial Sequence
<220>
   <223> D-Peptid (TD28ri)
<400> 2
   Pro Pro Tyr Tyr Leu Arg Met Gln Leu Ser Thr Thr
   1               5                   10
<210> 3
   <211> 12
   <212> PRT
   <213> Artificial Sequence
<220>
   <223> D-Peptid (Apt)
<400> 3
   Ala Pro Thr Leu Leu Arg Leu His Ser Leu Gly Ala
   1               5                   10
<210> 4
   <211> 12
   <212> PRT
   <213> Artificial Sequence
<220>
   <223> D-Peptid (Knt)
<400> 4
   Lys Asn Thr Pro Gln His Arg Lys Leu Arg Leu Ser
   1               5                   10

## Beschreibung

Die Erfindung betrifft Peptide zur Behandlung und Frühdiagnose von Morbus Alzheimer und anderen Tauopathien.

### Stand der Technik

Die Alzheimersche Demenz (AD) ist durch die pathologische Aggregation zweier Proteine, dem Aβ-Peptid (Amyloid-Beta-Peptid, A-Beta-Peptid) und dem Tau-Protein, gekennzeichnet. Beiderlei Arten von Proteinaggregaten gelten als neurotoxisch, während die jeweiligen Monomere nicht toxisch sind bzw. wichtige Funktionen in Wirbeltierzellen inne haben.

Das Tau-Protein bindet in Wirbeltier-Zellen an stützende Zytoskelett-Proteine (Mikrotubuli) und reguliert deren Zusammenbau. Neurodegenerative Erkrankungen mit Ablagerungen des Tau-Proteins werden in der Gruppe der Tauopathien zusammengefasst. Die bekannteste Tauopathie ist Morbus Alzheimer. Allerdings kommen pathologische Tau-Aggregate auch in 17 anderen Krankheiten, wie z. B. der Frontotemporalen Demenz und der Parkinsonschen Krankheit, vor. Zum Teil ist hier mutiertes Tau-Protein pathologisches alleiniges Hauptmerkmal.

Im menschlichen Zentralnervensystem lassen sich verschiedene Isoformen des Tau-Proteins nachweisen. Tau-Proteine können sich zusammenlagern und abnorme "gepaarte helikale Filamente" (PHF) bilden. Als Folge dieser pathologischen Veränderungen können die PHF-Ablagerungen als neurofibrilläre Bündel in den Gehirnen von Alzheimer-Patienten beobachtet werden. Da aggregierte Tau-Proteine nicht mehr funktionell sind, ist der Aufbau der Mikrotubuli gestört.

Kleine Moleküle, die die Aggregation des Tau-Proteins verhindern sollen, wurden bereits beschrieben. Die Anzahl ist jedoch gering im Vergleich zu Molekülen, die die Aβ-Aggregation verhindern oder rückgängig machen sollen. In letzter Zeit wurde in der Literatur stärker auf das Tau-Protein und dessen Zusammenspiel mit dem Aβ-Peptid hingewiesen, da die Therapie von Alzheimer unter alleiniger Fokussierung auf Aβ-Aggregate möglicherweise nicht den gewünschten Erfolg bringen kann. Beispielsweise führte eine Reduktion des Plaque-Gehaltes durch eine Anti-Aβ-Immuntherapie nicht zu den erwarteten Verbesserungen der kognitiven Leistungen der behandelten Patienten. Viele Wissenschaftler schreiben Aβ mittlerweile sogar eine neuroprotektive Wirkung zu.

Tau-Aggregationsinhibitoren und -Modulatoren können möglicherweise bestehende therapeutische Versuche komplementieren. Auch wenn bis heute nicht bekannt ist, was die Tau-Aggregations-Kaskade auslöst, besteht eine enge Korrelation zwischen der Anzahl an neurofibrillären Bündeln in den Neuronen, der zellulären Degeneration und den Demenzsymptomen.

Vorläufige Daten aus klinischen Studien der Phase 2 belegen, dass die Inhibition der Tau-Aggregation sinnvoll für eine Therapie der AD ist. Rhodaninbasierte Inhibitoren führten zu einer klaren Verringerung der Tau-Aggregation-basierten Zytotoxizität im Zytosol von neuronalen Zellmodellen. AL-108, die intranasale Formel für das aus acht Aminosäuren bestehende NAP-Peptid, zeigte in klinischen Studien der Phase IIa positiven Einfluss auf die Gedächtnisleitung von Patienten mit aMCI ("amnestic mild cognitive impairment"). Für NAP konnte unter anderem eine direkte Einflussnahme auf die Hyperphoshorylierung von Tau gezeigt werden. Erste Studien belegen Erfolge nach Tau-basierter Immuntherapie von transgenen Mausmodellen. Tau-basierte Therapien werden in Zukunft eine immer größer werdende Rolle spielen, da Tau-Aggregate infektiöse Eigenschaften besitzen. Die pathologische Tau-Aggregation kann von Zelle zu Zelle weitergegeben werden. Außerdem zeigen Tauexprimierende Mausmodelle nach einer künstlichen Infektion mit Tau-Aggregaten eine deutlichere Pathologie.

Neben therapeutischen Komponenten werden dringend bessere Diagnoseverfahren für die AD benötigt. Bis heute gibt es verschiedene Substanzen, die an aggregiertes Aβ-Peptid binden. Nach der Inkorporation von Radionukliden in diese Substanzen werden bildgebende Verfahren wie Positronen-Emissions-Tomographie (PET) und Einzelphotonen-Emissions-Tomografie (SPECT) am lebenden Patienten möglich ("in vivo Imaging"). Da die Anzahl der Plaques wenig mit dem Fortschritt der AD korreliert, sind Substanzen, die mit hoher Spezifität an aggregiertes Tau binden, möglicherweise eine diagnostische Alternative, da das Auftreten neurofibrillärer Tau-Bündel mit dem Krankheitsfortschritt korreliert. Bisher wurden nur wenige Substanzen beschrieben, die in vitro selektiv an das aggregierte Tau-Protein binden. Quinolin und Benzimidazole-Derivate färbten selektiv neurofibrilläre Bündel in Hirnschnitten von verstorbenen AD-Patienten.

Nachteilig können bisher nur die Symptome der AD behandelt werden. Es gibt keine zugelassenen Medikamente, die die Krankheitsprozesse aufhalten oder rückgängig machen können. Die meisten der Substanzen, die für die Therapie der AD erforscht werden, fokussieren auf extrazelluläres Aβ, nicht aber auf die Tau-Pathologie oder, vorzugsweise, auf beides.

Es gibt keine Sonden für das in-vivo-imaging, die speziell an Tau-Aggregate binden und diese sichtbar machen. Da Tau-Aggregate in der Krankheitsgeschichte eine so wichtige und frühe Rolle spielen, ist genau dieses wünschenswert. Im Gegensatz zu Aβ-Plaques korreliert die Menge an Tau-Ablagerungen im Gehirn mit dem Krankheitsfortschritt.

### Aufgabe und Lösung der Erfindung

Aufgabe der Erfindung ist es Peptide bereit zu stellen, die
A) eine ursächliche Therapie von Morbus Alzheimer oder anderer Tauopathien durch das Verhindern der Bildung von toxischen Tau-Aggregaten oder deren Enttoxifizierung ermöglichen bzw. die
B) eine Diagnose der Alzheimerschen Demenz oder anderer Tauopathien durch Verwendung der Peptide als Sonde für ein in vivo Imaging-Verfahren ermöglichen.

Die Aufgabe der Erfindung wird gelöst durch die Peptide gemäß Patentanspruch 1 sowie deren Verwendung. Die Erfindung wird auch gelöst durch das Verfahren, durch das KIT und durch die Zusammensetzung nach einem der nebengeordneten Patentansprüche. Vorteilhafte Ausgestaltungen ergeben sich aus den jeweils darauf zurückbezogenen Patentansprüchen.

### Beschreibung der Erfindung

Gemäß der vorliegenden Erfindung wird die Aufgabe der Erfindung durch ein Peptid enthaltend eine Aminosäuresequenz, die an das Volllängen-Tau-Protein bindet, gelöst. Die aus D-Aminosäuren aufgebaute Aminosäuresequenz des Peptids ist dabei ausgewählt aus SEQ ID NO: 4 (knt) und Polymeren der SEQ ID NO:4.

Im Sinne der Offenbarung bedeutet "im Wesentlichen aus D-Aminosäuren", dass die einzusetzenden Monomere mindestens 60 % bevorzugt 75 %, 80 %, besonders bevorzugt 85 %, 90 %, 95 %, insbesondere 96 %, 97 %, 98 %, 99 %, 100 % aus D-Aminosäuren aufgebaut sind. D-Aminosäuren verursachen vorteilhaft eine geringere Immunantwort als die entsprechenden L-Aminosäuren.

Ein Polymer im Sinne der Erfindung ist gebildet aus 2, 3, 4, 5, 6, 7, 8, 9, 10, 11, 12, 13, 14, 15, 16, 17, 18, 19, 20 oder mehr Monomeren der SEQ ID NO: 4 (Knt). Erfindungsgemäß sind die Polymere aus einem Monomer aufgebaut.

Monomere und Polymere werden im Folgenden als erfindungsgemäße Peptide bezeichnet. "Homologe Sequenzen" oder "Homologe" bedeutet im Sinne Offenbarung, dass eine Aminosäuresequenz eine Identität mit einer der oben genannten Aminosäuresequenz der Monomere von mindestens 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 % aufweist. Anstelle des Begriffs "Identität" werden in der vorliegenden Beschreibung die Begriffe "homolog" oder "Homologie" gleichbedeutend verwendet. Die Identität zwischen zwei Nukleinsäuresequenzen oder Polypeptidsequenzen wird durch Vergleich mit Hilfe des Programms BESTFIT basierend auf dem Algorithmus von Smith, T.F. und Waterman, M.S (Adv. Appl. Math. 2: 482-489 (1981)) berechnet unter Einstellung folgender Parameter für Aminosäuren: Gap creation penalty: 8 und Gap extension penalty: 2; und folgender Parameter für Nukleinsäuren: Gap creation penalty: 50 und Gap extension penalty: 3. Bevorzugt wird die Identität zwischen zwei Nukleinsäuresequenzen oder Polypeptidsequenzen durch die Identität der Nukleinsäuresequenz / Polypeptidequenz über die jeweils gesamte Sequenzlänge definiert, wie sie durch Vergleich mit Hilfe des Programms GAP basierend auf dem Algorithmus von Needleman, S.B. und Wunsch, C.D. (J. Mol. Biol. 48: 443-453) unter Einstellung folgender Parameter für Aminosäuren berechnet wird: Gap creation penalty: 8 und Gap extension penalty: 2; und die folgenden Parameter für Nukleinsäuren Gap creation penalty: 50 und Gap extension penalty: 3.

Zwei Aminosäuresequenzen sind im Sinne der vorliegenden Erfindung identisch wenn sie dieselbe Aminosäuresequenz besitzen.

Unter Homologe sind in einer Variante die entsprechenden retro-inversen Sequenzen der oben genannten Monomere zu verstehen. Mit dem Begriff "retro-inverse Sequenz" wird eine Aminosäuresequenz bezeichnet, die sich aus Aminosäuren in der enantiomeren Form zusammensetzt (invers: Chiralität des alpha-C-Atoms invertiert) und bei der zusätzlich die Sequenzreihenfolge zur ursprünglichen Aminosäuresequenz umgekehrt wurde (retro =rückwärts).

In einer weiteren Variante binden die erfindungsgemäßen Peptide auch an Teile des Tau-Proteins und/oder an das Volllängen-Tauprotein, insbesondere an aus dem Tau-Protein bestehende Aggregate.

Erfindungsgemäß handelt es sich um ein Peptid zur Verwendung in der Medizin, bevorzugt zur Behandlung der Morbus Alzheimer oder anderer Tauopathien.

In einer weiteren Variante handelt es sich um ein erfindungsgemäßes Peptid zur Hemmung der Fibrillenbildung bzw. Aggregation von Tau-Protein.

Die erfindungsgemäßen Peptide verhindern die (weiter)-Aggregation des Tau-Proteins durch Bindung an Monomere oder kleinere Oligomere, oder enttoxifizieren aus dem Tau-Protein gebildete Polymere, sowie Fibrillen, indem sie daran binden und so in nicht toxische Verbindungen überführen. Demgemäß ist Gegenstand der vorliegenden Erfindung auch eine Verwendung zur Enttoxifizierung von aus dem Tau-Proteingebildeten Polymeren oder Fibrillen. Gegenstand der Erfindung sind in einer Ausführung auch erfindungsgemäße Peptide, die mit einer weiteren Substanz verknüpft sind. Bei der Verknüpfung handelt es sich im Sinne der Erfindung um eine chemische Bindung wie sie in Römpp Chemie Lexikon, 9. Auflage, Band 1, Seite 650 ff, Georg Thieme Verlag Stuttgart definiert ist, bevorzugt um eine Hauptvalenz Bindung, insbesondere eine kovalente Bindung.

Bei den Substanzen handelt es sich in einer Variante um Arzneimittel oder Wirkstoffe, definiert gemäß Arzneimittelgesetz §2 beziehungsweise §4 (19), Stand September 2012.

In einer Alternative sind Wirkstoffe therapeutisch aktive Stoffe, die als arzneilich wirksame Stoffe verwendet werden. Bevorzugt werden Entzündungshemmer eingesetzt.

Bei den Substanzen handelt es sich in einer weiteren Variante um Verbindungen, die die Wirkung der erfindungsgemäßen Peptide verstärken. Das können auch andere Peptide sein, die z. B. an Tau oder Aβ binden.

In einer Alternative sind solche Verbindungen Aminopyrazol und/oder Aminopyrazolderivate. Aminopyrazolderivate im Sinne der Erfindung ist 3-Aminopyrazol-5-carbonsäure oder 3-Nitropyrazol-5-carbonsäure sowie alle Abkömmlinge, in denen die heterocyclische CH-Gruppe gegen -CR- oder -N oder -O- oder -S- ausgetauscht wurde, sowie alle daraus abgeleiteten peptidischen Dimere, Trimere oder -Tetramere, bevorzugt Aminopyrazol-Trimer.

In einer weiteren Alternative handelt es sich um Verbindungen, die die Löslichkeit der Peptide und/oder die Passage durch die Blut-Hirn-Schranke verbessern.

In einer Alternative haben die Peptide erfindungsgemäß jede beliebige Kombination von mindestens zwei oder mehr Merkmalen der oben beschriebenen Varianten, Ausführungen und/oder Alternativen.

Gegenstand der Erfindung ist auch ein erfindungsgemäßes Peptid zur Bindung an aggregierte Tau-Proteine.

Weiterer Gegenstand der Erfindung ist ein Verfahren zur Herstellung des erfindungsgemäßen Peptids durch Peptidsynthese, wie zum Beispiel dem Fachmann bekannt, organische Synthesemethoden für beliebige Verbindungen mit geringem Molekulargewicht (lowmolecular weight compounds) und/oder Mutagenese und rekombinante Herstellung.

Die Erfindung betrifft ferner auch eine Zusammensetzung, enthaltend das oder die erfindungsgemäße(n) Peptid(e), insbesondere zur Behandlung von Morbus Alzheimer oder anderer Tauopathien.

Gegenstand der vorliegenden Erfindung ist ferner eine Zusammensetzung enthaltend das oder die erfindungsgemäße(n) Peptid(e), insbesondere zur Verhinderung von toxischen Tau-Proteinaggregaten.

Die erfindungsgemäße "Zusammensetzung" kann z. B. ein Impfstoff, ein Medikament (z. B. in Tablettenform), eine Injektionslösung, ein Nahrungs- oder Nahrungsergänzungsmittel sein, enthaltend das erfindungsgemäße Peptid in einer aufgrund des Fachwissens herzustellenden Formulierung.

Die Erfindung betrifft ferner auch ein KIT enthaltend das erfindungsgemäße Peptid.

In einem solchen KIT können das oder die erfindungsgemäße(n) Peptid(e) in Behältern gegebenenfalls mit/in Puffern oder Lösungen verpackt sein. Alle Komponenten des KIT können in demselben Behälter oder getrennt voneinander verpackt sein. Ferner kann das KIT Anweisungen für dessen Gebrauch enthalten. Ein solches KIT kann beispielsweise die erfindungsgemäßen Peptide in einer Injektionsflasche mit Stopfen und/oder Septum enthalten. Ferner kann darin beispielsweise auch eine Einmal-Spritze enthalten sein.

Ein weiterer Gegenstand der vorliegenden Erfindung ist die Verwendung des erfindungsgemäßen Peptids als Sonde zur Identifizierung, qualitativen und/oder quantitativen Bestimmung von Tau-Proteinen, vorzugsweise Tau-Proteinaggregaten oder Fibrillen.

Gegenstand der vorliegenden Erfindung ist auch eine Sonde, enthaltend das oder die erfindungsgemäße(n) Peptid(e) zur Identifizierung, qualitativen und/oder quantitativen Bestimmung von Tau-Protein, vorzugsweise Proteinaggregaten oder Fibrillen.

Solche Sonden sind von großer Bedeutung, da damit eine frühe Diagnose der AD möglich wird. Mit der Frühdiagnose kann der Krankheit schon in einem sehr frühen Stadium entgegengewirkt werden.

Solche molekularen Sonden enthalten das erfindungsgemäße Peptid und gegebenenfalls Farbstoffe, Fluoreszenzfarbstoffe, radioaktive Isotope, (PET etc.), Gadolinium (MRI), sowie alternative Stoffe geeignet für die Bildgebung der Sonden und können den Patienten z. B. intravenös injiziert werden. Nach Passage über die Bluthirnschranke können die Sonden an Tau-Protein und/oder Ablagerungen hieraus binden. Die so markierten Tau-Proteine und/oder Ablagerungen hieraus können mittels bildgebender Verfahren wie z. B. SPECT, PET, CT, MRT, Protonen-MR-Spektroskopie und so weiter sichtbar gemacht werden.

Ferner betrifft die Erfindung auch die Verwendung des Peptids zur Verhinderung von Tau-Aggregaten und/oder Tau-Fibrillen (Bündel).

Das erfindungsgemäße Peptid wird auch zur Enttoxifizierung von toxischen Tau-Aggregaten verwendet. Insbesondere wird es verwendet, um an Tau-Protein und/oder deren Aggregate zu binden.

Weiterer Gegenstand ist die Verwendung des erfindungsgemäßen Peptids als Therapeutikum zur Behandlung von Morbus Alzheimer oder anderer Tauopathien.

Die erfindungsgemäßen Peptide binden besonders gut an Tau-Protein und Teile hiervon, bevorzugt an Tau-Aggregate und Fibrillen.

In einer Ausführung der Offenbarung besitzen die Peptide durch angefügte Sequenzen und Arginine eine erhöhte Bluthirnschrankengängigkeit und/oder Löslichkeit.

Die erfindungsgemäßen Peptide, hemmen die Fibrillenbildung/Aggregation von Tau-Protein sehr effizient.

Ferner binden die erfindungsgemäßen Peptide an Tau-Protein, wodurch amorphe Aggregate gebildet werden - wie durch dynamische Lichtstreuung bewiesen - die im ThT-Test negativ sind.

Weiterer Gegenstand der Offenbarung ist die Verwendung der erfindungsgemäßen Peptide in einem Verfahren zur Behandlung (in vitro, ex vivo) von Blut, Blutprodukten und/oder Organen, dadurch gekennzeichnet, dass das Blut, die Blutprodukte und/oder Organe dem menschlichen oder tierischen Körper entnommen werden und Tau-Proteine entfernt und/oder enttoxifiziert werden.

### Ausführungsbeispiele

Im Weiteren wird die Erfindung an Hand von Ausführungsbeispielen näher charakterisiert, ohne dass es hierdurch zu einer Einschränkung der Erfindung kommen soll.

### Selektion Tau-bindender Peptide:

Es wurden in einer Spiegelbild-Phagenselektion D-enantiomere Peptide selektioniert, die an das aggregierte Tau-Protein binden.

Es konnten mehrere D-enantiomere Peptide generiert werden, die sowohl an das aggregierte Tau-Teilpeptid als auch an das aggregierte Volllängen-Tau binden und deren Aggregation inhibieren bzw. zu einer Bildung amorpher Aggregate führen.

Bei Spiegelbild-Phagendisplay-Selektionen werden die Selektionsrunden gegen ein D-Enantiomer als Target durchgeführt, das synthetisch generiert werden muss. Da dies für das Volllänge Tau-Protein (441 Aminosäuren) nicht möglich ist, wurden die Selektionen gegen das D-Enantiomer eines 6-mer-Tau-Fragments (PHF-6) durchgeführt, welches in den Reapeats R2 und R3 von Tau vorkommt und verschiedenen Studien zufolge den Kern der neurofibrillären Bündel bildet (von Bergen, M., Assembly of T protein into Alzheimer paired helical filaments depends on a local sequence motif (306VQIVYK311) forming β structure PNAS, 1999. 97(10): p. 5129-5134; Miller, M., Synergistic Interactions between Repeats in Tau Protein and Aβ Amyloids May Be Responsible for Accelerated Aggregation via Polymorphic States. 5172-5181, 2011. 50(23): p. 5172-5181.). Da aggregiertes Material als Ziel für die Selektion dienen sollte, monomeres Tau-Protein hat wichtige physiologische Funktionen und darf in vivo nicht inhibiert werden, wurde ausschließlich fibrilläres Material zur Selektion eingesetzt. Überprüft wurde die Güte der Aggregation mittels Thioflavin T (ThT). ThT ist ein Fluoreszenzfarbstoff, der an ß-Faltblattreiche Strukturen verschiedener amyloider Proteine bindet und nach der Anregung bei 440 nm fluoresziert. Die Emission kann auf diese Weise mit dem relativen Fibrillengehalt in der Probe korreliert werden. ThT-Tests werden zur Messung der Fibrillisation von Aβ und Tau-Protein genutzt und vor allem bei Liganden verwendet, um eine mögliche inhibitorische Wirkung dieser auf die Aß- bzw. Tau-Aggregation nachzuweisen.

Für die folgenden Selektionen wurde ein Phagendisplay-Kit der Firma Biolabs verwendet. Parallel wurde ein reguläres Phagendisplay zur Selektion von L-Peptiden durchgeführt. Hier kann die Gewinnung von Bindemotiven nach Datenbankrecherchen Rückschlüsse auf bisher unbekannte Bindepartner in vivo liefern. Des Weiteren können Bindepartner später als D-Peptide oder retro-inverso-peptide getestet werden. Nach der Selektion wurden angereicherte Phagen vereinzelt und es wurden Einzelphagen-ELISA durchgeführt. Phagen, die im Einzelphagen-ELISA eine deutliche Bindung an PHF-6-Fibrillen, aber nicht an das leere Well vorwiesen, wurden amplifiziert, gefällt und die DNA wurde isoliert und sequenziert. Die D-Peptide Apt und Knt stammen aus Selektionen gegen D-PHF-6. Gegen L-PHF-6 wurde ein Peptid mit der Sequenz TTSLQMRLYYPP selektioniert, welches von uns auch in D-enantiomerer Form (TD28) und als retro-inverso-Peptid (TD28ri) untersucht wurde.

Folgende Peptidsequenz löst insbesondere die Aufgabe der Erfindung:
Knt: kntpqhrklrls

Während TD28 und TD28ri Tau-Fibrillen in große amorphe Aggregate umwandeln und somit möglicherweise detoxifizieren, löst Knt bestehende Tau-Fibrillen auf.

Es wurde bereits ein D-enantiomeres Peptid beschrieben, welches an das Tau-Fragment PHF-6 bindet. Es wurde durch in Silico-Methoden gefunden (Sequenz: tlkivw, Sievers et al., Nature. 2011 Jun 15; 475(7354):96-100. doi: 10.1038/nature101 54; US 2010/0204085 A1). In unseren Versuchen hemmte dieses Peptid die Aggregation von PHF-6 ähnlich gut wie die von uns entwickelten Peptide, nicht aber die Aggregation des Volllängen-Tauproteins.

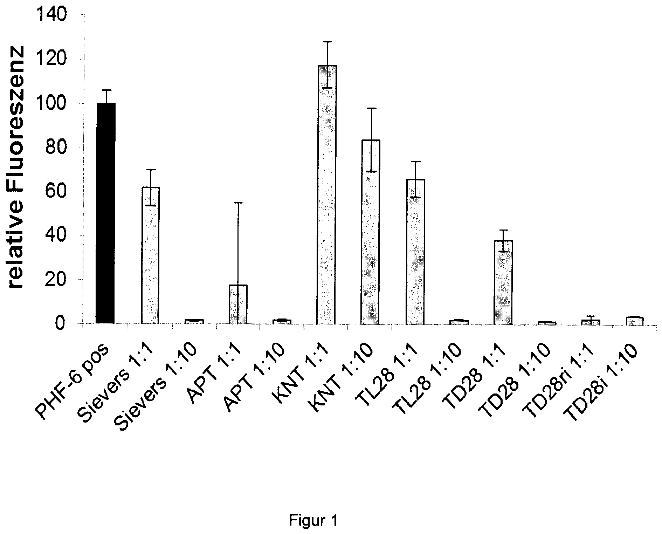

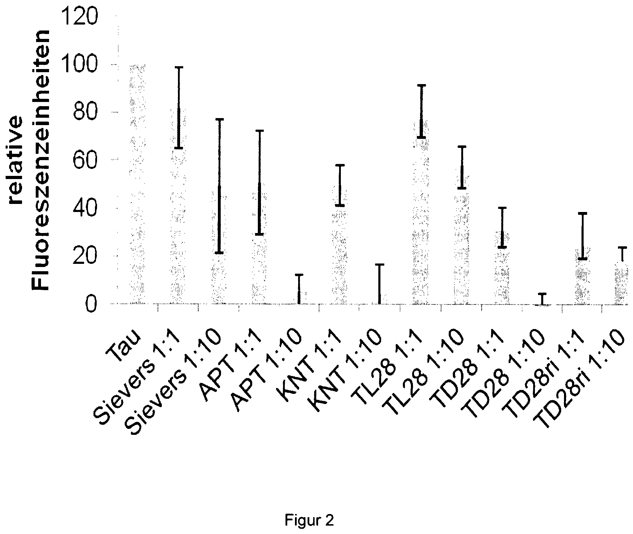

Charakterisierung der inhibitorischen Eigenschaften der Tau bindenden Peptide:
Im nächsten Schritt wurde der Einfluss der Tau-bindenden Peptide auf dessen Aggregation untersucht. Dazu wurde der oben bereits beschriebene ThT-Test verwendet. Die Untersuchungen wurden zunächst mit dem zur Selektion benutzten PHF-6-Fragment durchgeführt. Als Kontrolle wurde ein D-enantiomeres Peptid verwendet, das nach Beginn unserer Arbeiten als PHF-6-bindendes und Aggregations-inhibierendes Peptid beschrieben wurde. Es wurde durch in silico-Methoden generiert (Sievers, K.. Structure-based design of non-natural amino-acid inhibitors of amyloid fibril formation. Nature, 2011. 475: p. 96-100) und auf maximale Wirkung optimiert. Die generierten Peptide wurden in Verhältnissen von 1:1 und 10:1 zu PHF-6 eingesetzt. Parallel wurden die Peptide ohne PHF-6 getestet (Negativkontrolle auf Eigenfibrillisation) und PHF-6 ohne Tau-bindende Peptide (Positivkontrolle, maximale Aggregation). Wie in Figur 1 gezeigt, bewirkten alle Peptide eine Dosis-abhängige Reduktion der Fibrillenbildung von PHF-6. Einige der Peptide zeigten eine ähnlich starke Inhibierung der Fibrillisation wie das aus der Literatur bekannte von Sievers et al. beschriebene Peptid. Anschließend wurde untersucht, ob die Peptide nicht nur die Fibrillisation von PHF-6, sondern auch die des Volllängen-Tau-Proteins inhibierten. Wie in Figur 2 zu sehen, inhibierten alle untersuchten Peptide die Fibrillisation des Volllängen-Tau-Proteins. Alle selektionierten Peptide inhibierten danach die Fibrillisation besser als das von Sievers selektionierte Peptid, welches in den meisten Ansätzen gar keine Wirkung zeigte.

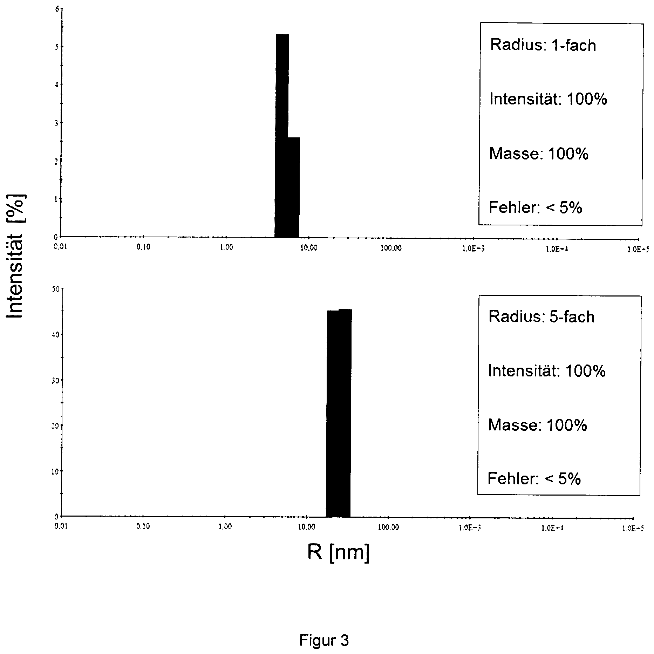

Da für die selektionierten Peptide eine starke Inhibition der Fibrillisation von Tau beobachtet werden konnte, wurden zusätzlich Messungen mit dynamischer Lichtstreuung (DLS) durchgeführt, um die Aggregatbildung im Allgemeinen, unabhängig von der Sekundärstruktur der Partikel, zu verfolgen. Hierfür wurden 1µg/ml Tau-Volllängen-Protein mit 100 µM Arachidonsäure und das jeweilige Peptid im Verhältnis 1:10 untersucht. Als Kontrolle wurde Tau ohne Peptid und ohne Zugabe von Arachidonsäure vermessen. Letzteres diente als Negativkontrolle, da Tau unter den angelegten Versuchsbedingungen ohne Arachidonsäure nicht aggregierte (Figur 3). Es zeigte sich, dass ein Teil der Peptide die Aggregation von Tau über den gemessenen Versuchszeitraum verhinderte, während andere Peptide (TD28 und TD28ri) zu der Bildung sehr großer Aggregate führten (siehe Tabelle 1). Die Tatsache, dass bei den gleichen Konzentrationsverhältnissen im ThT-Test keine ThT-positiven Fibrillen detektiert werden konnten, deutet darauf hin, dass es sich bei den Peptid-induzierten Partikein nicht um fibrilläre Aggregate handelte, sondern um amorphe und wahrscheinlich nichttoxische Aggregate. Ein ähnlicher Mechanismus wurde bereits für das Peptid D3 aus EP 1 379 546 B1 gezeigt, das toxische Aβ-Oligomere in nicht toxische, amorphe Aggregate umwandelt. Das von Sievers et al. selektionierte Peptid zeigte keinen Effekt auf die Aggregation des Volllängen-Tau-Proteins.

**Tabelle 1:Zusammengefasste Ergebnisse der DLS-Messungen mit Tau und verschiedenen Peptiden. Tau ohne Zugabe von Arachidonsäure (AA) zeigte keine Tendenz zur Aggregation. Inkubation von Tau mit AA führte zur Aggregation und einer 5-fachen Steigerung der Partikelgröße.**

| Probe | Partikelgröße Tag 1 | Masse Tag 1 | Partikelgröße Tag 8 | Masse Tag 8 |
|---|---|---|---|---|
| Tau | 1-fach | ≥ 99 % | 1-fold | ≥ 98 % |
| Tau + AA | 5-fach | 100 % | 4-fold | 100 % |
| Tau + AA + Sievers | 6-fach | 100 % | 6-fold | ≥ 98 % |
| Tau + AA + APT | 1-fach | ≥ 99 % | 1-fold | ≥ 98 % |
| Tau + AA + KNT | 1-fach | ≥ 99 % | 1-fold | ≥ 99 % |
| Tau + AA + LPS | 1-fach | ≥ 99 % | 1-fold | ≥ 99 % |
| Tau + AA + D3 | 1-fach | 100 % | 1-fold | 100 % |
| Tau + AA + TL28 | 5-fold | ≥ 99 % | 4-fold | 100 % |
| Tau + AA + TD28 | 42-fold | 100 % | 48-fold | 100 % |
| Tau + AA + TD28ri | 55-fold | 100% | 44-fold | 100% |

Es zeigen:
- Figur 1: ThT-Aggregationstest von PHF-6 zur Quantifizierung des relativen Fibrillengehalts in Gegenwart verschiedener PHF-6-bindender Peptide. Die Konzentration von PHF-6 betrug 25 µM, die Peptide wurden im Verhältnis von 1:1 und 1:10 (PHF-6:Peptid) sowie als Kontrolle einzeln in einer 25 µM Konzentration gemessen (nicht gezeigt). Die Fluoreszenz von 25 µM PHF-6 nach zwei Stunden Inkubation wurde als 100 % gesetzt und die Werte und Standardabweichungen der übrigen Inkubationen sind als prozentuale Anteile dieses Maximalwerts angegeben.
- Figur 2: ThT-Aggregationstest von Tau zur Quantifizierung des relativen Fibrillengehalts in Gegenwart verschiedener PHF-6-bindender Peptide und 100 µM Arachidonsäure. Die Konzentration von Tau betrug 5 µM, die Peptide wurden im Verhältnis von 1:1 und 1:10 (Tau:Peptid) hinzugegeben. Die Fluoreszenz von 5 µM Tau nach 24 Stunden Inkubation wurde als 100 % gesetzt und die Werte und Standardabweichungen der übrigen Inkubationen sind als prozentuale Anteile dieses Maximalwerts angegeben.
- Figur 3: DLS-Messungen von Tau nach 6-stündiger Inkubation ohne (oben) und mit (unten) Zugabe von 100 µM Arachidonsäure. Tau wurde in PBS-Puffer zu einer Konzentration von 1 mg/ml gelöst. DLS wurde mit einer Aquisitionszeit von 5 s für 3 min bei Raumtemperatur durchgeführt.

## Patentansprüche

1. Peptid, enthaltend mindestens eine aus D-Aminosäuren aufgebaute Aminosäuresequenz, welche an das Volllängen-Tau-Protein bindet, ausgewählt aus SEQ ID NO: 4 und Polymere der SEQ ID NO: 4.

2. Peptid nach Anspruch 1 **dadurch gekennzeichnet, dass** dieses mit einer weiteren Substanz verknüpft ist.

3. Peptid nach einem der vorangehenden Ansprüche zur Verwendung in der Medizin.

4. Peptid nach einem der Ansprüche 1 und 2 zur Verwendung in der Behandlung der Morbus Alzheimer oder anderer Tauopathien.

5. Peptid nach einem der Ansprüche 1 und 2 zur Verwendung bei der Hemmung der Fibrillenbildung von Tau-Protein.

6. Peptid nach einem der Ansprüche 1 und 2 zur Verwendung bei der Bindung an aggregiertes Tau-Protein.

7. Verfahren zur Herstellung eines Peptids nach einem der Ansprüche 1 und 2, **dadurch gekennzeichnet, dass** das Peptid durch Peptid-Synthese oder Mutagenese hergestellt wird.

8. KIT, enthaltend ein Peptid nach einem der Ansprüche 1 und 2.

9. Zusammensetzung enthaltend ein Peptid nach einem der Ansprüche 1 und 2.

10. Peptid nach einem der Ansprüche 1 und zur Verwendung als Sonde zur Identifizierung, quantitativen und/oder qualitativen Bestimmung von Tau-Protein, dessen Aggregaten und/oder Tau-Fibrillen.

11. Peptid nach einem der Ansprüche 1 und 2 zur Verwendung bei der Verhinderung von Tau-Protein-Aggregaten.

12. Peptid nach einem der Ansprüche 1 und 2 zur Verwendung bei der Enttoxifizierung oder Verhinderung von toxischen Tau-Protein-Aggregaten.

13. Peptid zur Verwendung nach Anspruch 12, **gekennzeichnet durch** eine Verwendung bei der Tau-Protein-Aggregate mit dem Peptid nach einem der Ansprüche und 2 amorphe, nicht toxische Aggregate bilden.

## Claims

1. Peptide, containing at least one amino acid sequence made of D-amino acids, which binds to full-length tau protein, selected from SEQ ID NO: 4 and polymers of SEQ ID NO: 4.

2. Peptide according to claim 1, **characterised in that** this is combined with a further substance.

3. Peptide according to one of the previous claims for use in medicine.

4. Peptide according to one of claims 1 and 2 for use in the treatment of Morbus Alzheimer or other tauopathies.

5. Peptide according to one of claims 1 and 2 for use in inhibiting the fibril formation of tau protein.

6. Peptide according to one of claims 1 and 2 for use in binding to aggregated tau protein.

7. Method for producing a peptide according to one of claims 1 and 2, **characterised in that** the peptide is produced through peptide synthesis or mutagenesis.

8. KIT, containing a peptide according to one of claims 1 and 2.

9. Compound containing a peptide according to one of claims 1 and 2.

10. Peptide according to one of claims 1 and 2 for use as a probe for identification, quantitative and/or qualitative determination of tau protein, its aggregates and/or tau fibrils.

11. Peptide according to one of claims 1 and 2 for use in preventing tau protein aggregates.

12. Peptide according to one of claims 1 and 2 for use in detoxifying or preventing toxic tau protein aggregates.

13. Peptide for use according to claim 12, **characterised by** use, in which tau protein aggregates form amorphous, non-toxic aggregates with the peptide according to one of claims 1 and 2.

## Revendications

1. Peptide, contenant au moins une séquence d'acides aminés, constituée d'acides aminés D, qui se fixe à la protéine tau de longueur complète, choisie dans l'identification de séquence n° 4 et des polymères de l'identification de séquence n° 4.

2. Peptide suivant la revendication 1, **caractérisés en ce que** ceux-ci sont combinés à une autre substance.

3. Peptide suivant l'une des revendications précédentes, pour l'utilisation en médecine.

4. Peptide suivant l'une des revendications 1 et 2, à utiliser dans le traitement de la maladie d'Alzheimer ou d'autres tauopathies.

5. Peptide suivant l'une des revendications 1 et 2, à utiliser dans l'inhibition de la fibrillation de la protéine tau.

6. Peptide suivant l'une des revendications 1 et 2, à utiliser dans la fixation d'une protéine tau agrégée.

7. Procédé de préparation d'un peptide suivant l'une des revendications 1 et 2, **caractérisés en ce que** l'on prépare le peptide par synthèse peptidique ou par mutagénèse.

8. Trousse contenant un peptide suivant l'une des revendications 1 et 2.

9. Composition contenant un peptide suivant l'une des revendications 1 et 2.

10. Peptide suivant l'une des revendications 1 et 2, à utiliser comme sonde pour l'identification, la détermination quantitative et/ou qualitative de la protéine tau, de ses agrégats et/ou des fibrilles de tau.

11. Peptide suivant l'une des revendications 1 et 2, à utiliser dans la prévention d'agrégats de la protéine tau.

12. Peptide suivant l'une des revendications 1 et 2, à utiliser dans la détoxification ou la prévention d'agrégats toxiques de la protéine tau.

13. Peptide à utiliser suivant la revendication 1 ou 2, **caractérisés par** une utilisation dans laquelle des agrégats de protéine tau forment, avec le peptide suivant l'une des revendications 1 et 2, des agrégats amorphes non toxiques.
